# EUROPEAN PATENT APPLICATION

(11) **EP 4 822 709 A1**
(43) Date of publication of application: **14.10.2026**
(21) Application number: 25169536.7
(22) Date of filing: 09.04.2025
(51) Int. Cl.: G16H 50/20, G16H 50/70, A61B 5/0205, A61B 5/08, A61B 5/00

(54) **SYSTEM AND METHOD FOR MONITORING A SUBJECT**

(71) Applicant: Koninklijke Philips N.V., 5656 AE Eindhoven (NL)
(72) Inventor: PAPINI, Gabriele, 5656 AG Eindhoven (NL); TEN KATE, Warner Rudolph Theophile, 5656 AG Eindhoven (NL); COX, Lieke Gertruda Elisabeth, 5656 AG Eindhoven (NL)
(74) Representative: Philips Intellectual Property & Standards

(57) **Abstract**

A system and method for monitoring a subject. A first set of physiological data is obtained and processed, using a trained machine-learning algorithm, to generate a second set of physiological data. The trained machine-learning algorithm is trained using subject-specific training data comprising physiological data corresponding to the first set of physiological data and physiological data corresponding to the second set of physiological data.

## Description

### FIELD OF THE INVENTION

The invention relates to the field of health monitoring.

### BACKGROUND OF THE INVENTION

Monitoring a subject's health typically involves recording several physiological signals. The number (and type) of physiological signals recorded depends on the subject and their health status.

For instance, immediately after surgery, a subject may be monitored using a bedside monitor. Once the subject has progressed further in their recovery, the subject may be moved to a monitoring system that is less burdensome and restrictive, such as a telemetry monitor. A telemetry monitor typically measures only a subset of the physiological signals measured by a bedside monitor. For example, a bedside monitor may acquire a 12-lead electrocardiography signal (used to determine heart rate and to detect arrhythmias), a capnography signal (used to determine end tidal CO₂ and respiration rate) and a photoplethysmography signal (used to determine blood oxygen saturation), while a telemetry monitor may include only a 8-lead electrocardiography signal and a photoplethysmography signal. A model may then be used to derive a 12-lead electrocardiography signal and an ECG-based respiration rate from the 8-lead electrocardiography signal .

### SUMMARY OF THE INVENTION

The invention is defined by the claims.

According to examples in accordance with an aspect of the invention, there is provided a processing system for monitoring a subject, the processing system being configured to: obtain a first set of physiological data for the subject; and process the first set of physiological data, using a first trained machine-learning algorithm, to generate a second set of physiological data for the subject, wherein the first trained machine-learning algorithm is a machine-learning algorithm trained using subject-specific training data comprising physiological data corresponding to the first set of physiological data and physiological data corresponding to the second set of physiological data.

The inventors have recognized that the use of subject-specific training data to train the first machine-learning algorithm improves a reliability of the second set of physiological data.

The second set of physiological data may, for example, comprise one or more types of physiological signals and/or metrics that are not included in the first set of physiological data. In another example, the second set of physiological data may comprise physiological signals and/or metrics of a same type as the first set of physiological data, and the physiological signals and/or metrics in the second set of physiological data may have improved quality and/or additional (synthesized) data values compared to the physiological signals and/or metrics in the second set of physiological data.

In some examples, the subject-specific training data comprises historical physiological data for the subject.

The inventors have recognized that, in many applications in which a machine-learning algorithm is used to generate a second set of physiological data based on a first set of physiological data, physiological data corresponding to the second set of physiological data has often been acquired for the subject at an earlier period in time. For instance, a subject being monitored using a telemetry monitor may previously have been monitored using a bedside monitor; the data acquired by the bedside monitor may thus be used to train a machine-learning algorithm to generate data of the type(s) acquired by a bedside monitor using data acquired by the telemetry monitor.

In some examples, the subject-specific training data comprises physiological data for each of a plurality of individuals, wherein each of the plurality of individuals have a same one or more predefined characteristics as the subject.

The use of physiological data from individuals having one or more predefined characteristics in common with the subject to train the first machine-learning algorithm provides better results than a machine-learning algorithm trained on a general population. In some cases (for instance, when a current situation of the subject is significantly different to the situation in which suitable data for training the first machine-learning algorithm is acquired from the subject), a machine-learning algorithm trained using physiological data from individuals having one or more predefined characteristics in common with the subject may provide better results than a machine-learning algorithm trained using historical physiological data for the subject.

In some examples, the one or more predefined characteristics includes a medical condition.

The one or more predefined characteristics may additionally or alternatively include one or more of: a sex, an age, a medical condition, a clinical procedure undergone, a medication taken or prescribed, a type of monitoring used and/or a treatment performed.

In some examples, the processing system is further configured to determine a quality metric for the second set of physiological data.

The quality metric may, for example, be output by the first trained machine-learning algorithm, or a separate machine-learning algorithm may be trained to output the quality metric.

In some examples, the processing system is further configured to control a feedback unit to provide a first user-perceptible output in response to the quality metric failing to exceed a predetermined quality threshold.

In this way, the second set of physiological data may be identified as unreliable by a clinician.

In some examples, the processing system is further configured to: obtain one or more conditions under which the first set of physiological data is expected to result in an unreliable second set of physiological data; and control a feedback unit to provide a second user-perceptible output in response to at least one of the one or more conditions being met.

In this way, the second set of physiological data may be identified as unreliable by a clinician.

In some examples, the processing system is further configured to: obtain the subject-specific training data used to train the first machine-learning algorithm; compare the subject-specific training data with the first set of physiological data to determine a measure of difference; and control a feedback unit to provide a third user-perceptible output in response to the measure of difference exceeding a predetermined difference threshold.

In this way, the second set of physiological data may be identified as unreliable by a clinician.

In some examples, the processing system is further configured to, in response to a predetermined condition being met, process the first set of physiological data, using a second trained machine-learning algorithm, to generate the second set of physiological data, wherein the second trained machine-learning algorithm is a machine-learning algorithm trained using second training data that is less subject-specific than the subject-specific training data used to train the first trained machine-learning algorithm.

In this way, whichever machine-learning algorithm is predicted to give the better results may be used to generate the second set of physiological data. The predetermined condition may, for example, relate to time or an estimated quality of the second set of physiological data.

In some examples, the processing system is further configured to control a display device to display the second set of physiological data.

In some examples, the first set of physiological data comprises an 8-lead electrocardiography signal and the second set of physiological data comprises a 12-lead electrocardiography signal.

In some examples, the first set of physiological data comprises an electrocardiography signal and the second set of physiological data comprises a respiration rate.

In some examples, the first set of physiological data comprises a plethysmography signal and the second set of physiological data comprises a respiration rate.

According to examples in accordance with another aspect of the invention, there is provided a computer-implemented method for monitoring a subject, the computer-implemented method comprising: obtaining a first set of physiological data for the subject; and processing the first set of physiological data, using a first trained machine-learning algorithm, to generate a second set of physiological data for the subject, wherein the first trained machine-learning algorithm is a machine-learning algorithm trained using subject-specific training data comprising physiological data corresponding to the first set of physiological data and physiological data corresponding to the second set of physiological data.

There is also provided a computer program product comprising computer program code means which, when executed on a computing device having a processing system, cause the processing system to perform all of the steps of the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system for monitoring a subject, according to an embodiment of the invention; and
Fig. 2 illustrates a computer-implemented method for monitoring a subject, according to an embodiment of the invention.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system and method for monitoring a subject. A first set of physiological data is obtained and processed, using a trained machine-learning algorithm, to generate a second set of physiological data. The trained machine-learning algorithm is trained using subject-specific training data comprising physiological data corresponding to the first set of physiological data and physiological data corresponding to the second set of physiological data.

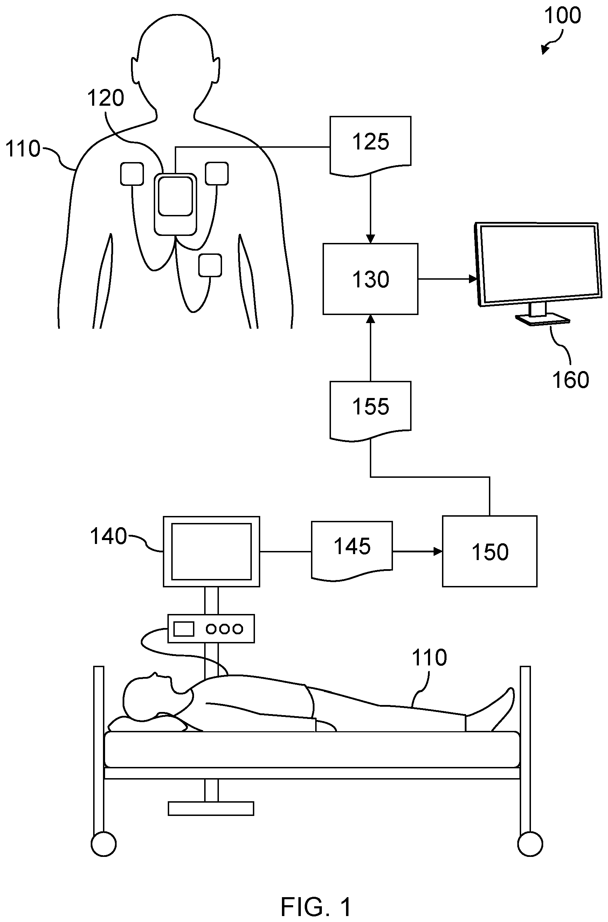

Fig. 1 illustrates a system 100 for monitoring a subject 110, according to an embodiment of the invention. The system 100 comprises a monitor 120 for acquiring physiological data for the subject, and a first processing system 130. The first processing system is, itself, an embodiment of the invention.

In Fig. 1, the monitor 120 is a telemetry monitor; however, it is to be understood that any monitor capable of acquiring physiological data for a subject, such as a monitor for measuring continuous vital signs, may be used. For example, the monitor may be a telemetry monitor, a bedside monitor, a smartwatch or a smart patch. The monitor may comprise one or more sensors for acquiring the physiological data; for instance, the monitor may comprise one or more of: electrocardiography electrodes, a photoplethysmography sensor, one or more accelerometers, and/or one or more cameras (which may be used to determine vital signs).

The first processing system 130 may be used to complete or augment a set of physiological data acquired by the monitor, in order to improve a clinical usefulness of the set of physiological data. For instance, where the monitor is unable to acquire one or more desired physiological signals, the first processing system may be used to synthesize at least one of the one or more desired physiological signals and/or one or more metrics derivable from the one or more desired physiological signals. For example, where the monitor is a telemetry monitor (as shown in Fig. 1), the first processing system may be used to synthesize one or more physiological signals and/or one or more metrics that would be obtainable using a bedside monitor. In another example (where the monitor may be any type of monitor, e.g. a bedside monitor), the first processing system may be used to synthesize missing data values and/or to replace low quality data with better quality data; this may be the case, for instance, where the first set of physiological data is affected by noise, artifacts and/or cable disconnection.

The first processing system 130 is configured to obtain a first set of physiological data 125. The first set of physiological data 125 is a set of physiological data acquired by the monitor 120, and may include any physiological signals acquired by the monitor and/or any metrics determined using one or more physiological signals acquired by the monitor. For instance, depending on the type of monitor used to acquire the first set of physiological data, the first set of physiological data may comprise one or more of: an electrocardiography signal (e.g. an 8-lead electrocardiography signal), a plethysmography signal, one or more non-invasive blood pressure measurements (e.g. one or more cuff pressure measurements), a respiration rate or waveform (e.g. an electrocardiography-derived respiration rate or waveform), and/or movement data (e.g. an accelerometer signal).

In Fig. 1, the first processing system obtains the first set of physiological data 125 directly from the monitor 120; however, it is to be understood that the first processing system may alternatively obtain the first set of physiological data from another source storing the first set of physiological data.

Having obtained the first set of physiological data 125, the first processing system 130 is configured to process the first set of physiological data, using a first trained machine-learning algorithm 155, to generate a second set of physiological data for the subject. In some examples, the first processing system 130 may be configured to continuously process the first set of physiological data as the first set of physiological data is acquired (e.g. the first processing system may be configured to generate the second set of physiological data in real-time or near real-time).

The second set of physiological data may comprise one or more physiological signals and/or one or more metrics; the one or more physiological signals and/or one or more metrics included in the second set of physiological data depends on the type(s) of physiological data included in the first set of physiological data and on the first trained machine-learning algorithm. For instance, depending on the first set of physiological data and the first trained machine-learning algorithm, the second set of physiological data may comprise one or more of: an electrocardiography signal (e.g. a 12-lead electrocardiography signal), a respiration rate or waveform, a continuous blood pressure signal, a core body temperature, a heart rate, any detected arrhythmia events and/or one or more motion-filtered physiological signals or metrics (e.g. a motion-filtered echocardiography signal, motion-filtered arrhythmia detection and/or a motion-filtered heart rate).

A machine-learning algorithm is any self-training algorithm that processes input data in order to produce or predict output data. Here, the input data comprises the first set of physiological data 125 and the output data comprises the second set of physiological data.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

A decision tree algorithm processes input data through a tree of nodes. In the tree of nodes, each successive node splits into two or more further nodes until reaching a terminal or end node. When performing the decision tree algorithm using the tree of nodes, at each node, a decision is made as to which further node to move to next based on the input data. The end node defines the outcome of the decision tree algorithm, and therefore the machine-learning algorithm.

Methods of training a machine-learning algorithm are well known. Typically, such methods comprise obtaining a training dataset, comprising training input data entries and corresponding training output data entries.

For some machine-learning algorithms, such as a neural network, training is performed by applying an initialized machine-learning algorithm to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

Other approaches for training machine-learning algorithms (e.g., decision trees) are known in the art. For instance, decision trees are often trained using a decision tree builder or learning techniques, such as those set out by Suthaharan, Shan, and Shan Suthaharan. "Decision tree learning." Machine Learning Models and Algorithms for Big Data Classification: Thinking with Examples for Effective Learning (2016): 237-269 or Ruggieri, Salvatore. "Yadt: Yet another decision tree builder." 16th IEEE International Conference on Tools with Artificial Intelligence. IEEE, 2004.

The first trained machine-learning algorithm 155 is a machine-learning algorithm that has been trained using subject-specific training data that comprises physiological data corresponding to the first set of physiological data and physiological data corresponding to the second set of physiological data. In other words, the subject-specific training data may comprise one or more training sets of physiological data, each training set including physiological data having a same one or more types as the first set of physiological data and physiological data having a same one or more types as the second set of physiological data. The physiological data in the subject-specific training data having a same one or more types as the first set of physiological data is used as training input data, while the physiological data in the subject-specific training data having a same one or more types as the second set of physiological data is used as training output data. Where the first trained machine-learning algorithm is used to synthesize missing data values or to replace low quality data with better quality data, the training input data may comprise physiological data having a same one or more types as the training output data.

The subject-specific training data may comprise historical physiological data for the subject and/or physiological data for each of a plurality of individuals having a same one or more predefined characteristics as the subject. The one or more predefined characteristics may, for example, include one or more of: a sex, an age, a medical condition, a clinical procedure undergone (e.g. a surgical procedure), medication taken or prescribed, a type of monitoring used, and/or a treatment performed. In some examples, the one or more predefined characteristics may include at least a medical condition; in other words, each of the plurality of individuals may have a same medical condition as the subject 110.

The subject-specific training data may be acquired using any suitable apparatus, i.e. any apparatus capable of acquiring physiological data having a same one or more types as the first set of physiological data and physiological data having a same one or more types as the second set of physiological data. For example, where the first machine-learning algorithm is used to synthesize missing data values, each training set in the subject-specific training data may be acquired using a monitor of a same or similar type to the monitor used to acquire the first set of physiological data. In examples in which the first machine-learning algorithm is used to synthesize one or more physiological signals and/or one or more metrics that cannot be acquired by the monitor used to acquire the first set of physiological data, each training set in the subject-specific training data may be acquired using a different type of monitor to the monitor used to acquire the first set of physiological data.

In Fig. 1, historical physiological data 145 for the subject 110 is acquired by a bedside monitor 140 (i.e. physiological data for the subject acquired by the bedside monitor at an earlier period than the period in which the first set of physiological data 125 is acquired). The historical physiological data 145 is obtained by a second processing system 150, which is configured to process the historical physiological data 145 to generate the subject-specific training data, and to train the first machine-learning algorithm 155 using the subject-specific training data. In this way, the first trained machine-learning algorithm 155 is trained to synthesize one or more physiological signals and/or one or more metrics that would be obtainable using a bedside monitor from physiological data acquired by the (telemetry) monitor 120.

In some examples, the first trained machine-learning algorithm may be trained by obtaining a generic machine-learning algorithm, which is trained using generic (i.e. not subject-specific) training data, and retraining the generic machine-learning algorithm using the subject-specific training data. The generic training data includes physiological data from individuals who do not share one or more characteristics with the subject 110. Retraining a generic machine-learning algorithm using the subject-specific training data enables the first machine-learning algorithm to be trained faster.

In Fig. 1, the first trained machine-learning algorithm 155 is trained by the second processing system 150, and is then obtained from the second processing system by the first processing system 130 in order to use the first trained machine-learning algorithm to process the first set of physiological data 125. However, it is to be understood that the first trained machine-learning algorithm may alternatively be trained by the processing system that is configured to use the first trained machine-learning algorithm to process the first set of physiological data to generate the second set of physiological data. For illustrative purposes, Fig. 1 shows the first processing system 130 obtaining the first trained machine-learning algorithm directly from the second processing system 150; it is to be understood that the first processing system may alternatively obtain the first trained machine-learning algorithm (whichever processing system is used to train the first trained machine-learning algorithm) from another source storing the first trained machine-learning algorithm (e.g. a memory unit).

Processing the historical physiological data 145 to generate the subject-specific training data may comprise removing data from or modifying the historical physiological data to generate the training input data and/or processing the historical physiological data to determine one or more metrics to be included in the training output data. As the skilled person will appreciate, how the historical physiological data is processed to generate the subject-specific training data depends on a desired application of the first trained machine-learning algorithm.

In some examples, the first trained machine-learning algorithm may be trained to synthesize missing data values in the first set of physiological data. To generate suitable subject-specific training data for training the trained machine learning algorithm, the historical physiological data may be processed to generate training input data in which some of the data values from the historical physiological data have been removed (while the complete set of historical physiological data may be included in the training output data). The training input data entries and corresponding training output data entries may each comprise a single type of physiological data (e.g. a single signal) and/or a set of physiological signals.

In some examples, the first trained machine-learning algorithm may be trained to replace low quality data with better quality data. To generate suitable subject-specific training data for training the trained machine learning algorithm, the historical physiological data may be processed to generate a low quality version of the historical physiological data, for example, to add synthesized artifacts and/or to increase noise. The training input data may then comprise the low quality version of the historical physiological data, while the training output data may comprise the historical physiological data (i.e. with the original quality).

In some examples, the first trained machine-learning algorithm may be trained to synthesize an electrocardiography signal from a higher number of leads using an electrocardiography signal from a lower number of leads. In other words, the first set of physiological data may comprise an electrocardiography signal from a lower number of leads, and the second set of physiological data may comprise an electrocardiography signal from a higher number of leads (e.g. the first set of physiological data may comprise an 8-lead electrocardiography signal and the second set of physiological data may comprise a 12-lead electrocardiography signal). The subject-specific training data may, for example, be generated using historical physiological data comprising an electrocardiography signal from the higher number of leads. The electrocardiography signal in the historical physiological data may be included in the training output data, and may be processed to generate an electrocardiography signal from the lower number of leads to be included in the training input data.

In some examples, the first trained machine-learning algorithm may be trained to synthesize a respiration rate (or respiration waveform) using an electrocardiography signal. In other words, the first set of physiological data may comprise an electrocardiography signal, and the second set of physiological data may comprise a respiration rate (or respiration waveform). The subject-specific training data may, for example, be generated using historical physiological data comprising an electrocardiography signal and a capnography signal. The electrocardiography signal in the historical physiological data may be included in the training input data, and the capnography signal may be processed to generate a respiration rate (or respiration waveform) to be included in the training output data.

In some examples, the first trained machine-learning algorithm may be trained to synthesize a respiration rate (or respiration waveform) using a plethysmography signal. In other words, the first set of physiological data may comprise a plethysmography signal, and the second set of physiological data may comprise a respiration rate (or respiration waveform). The subject-specific training data may, for example, be generated using historical physiological data comprising a plethysmography signal and a capnography signal. The plethysmography signal in the historical physiological data may be included in the training input data, and the capnography signal may be processed to generate a respiration rate (or respiration waveform) to be included in the training output data. Alternatively, the subject-specific training data may be generated using historical physiological data comprising a plethysmography signal and an electrocardiography signal. The plethysmography signal in the historical physiological data may be included in the training input data, and the electrocardiography signal may be processed to generate a respiration rate (or respiration waveform) to be included in the training output data. The electrocardiography signal may be processed to generate the respiration rate (or respiration waveform) using a separate trained machine-learning algorithm, which may have a similar or same architecture to the first trained machine-learning algorithm but that has been trained using different training data to output a respiration rate (or respiration waveform) based on an electrocardiography signal.

In some examples, the first trained machine-learning algorithm may be trained to synthesize a continuous blood pressure signal using a plethysmography signal. In other words, the first set of physiological data may comprise a plethysmography signal, and the second set of physiological data may comprise a continuous blood pressure signal. The subject-specific training data may, for example, be generated using historical physiological data comprising an invasively-acquired continuous blood pressure signal and a plethysmography signal. The plethysmography signal in the historical physiological data may be included in the training input data, and the invasively-acquired continuous blood pressure signal may be included in the training output data.

In some examples, the first trained machine-learning algorithm may be trained to synthesize a continuous blood pressure signal using a plethysmography signal and one or more non-invasive blood pressure measurements (e.g. acquired using a pressure cuff). In other words, the first set of physiological data may comprise a plethysmography signal and one or more non-invasive blood pressure measurements, and the second set of physiological data may comprise a continuous blood pressure signal. The subject-specific training data may, for example, be generated using historical physiological data comprising an invasively-acquired continuous blood pressure signal and a plethysmography signal. The plethysmography signal in the historical physiological data may be included in the training input data. The invasively-acquired continuous blood pressure signal may be included in the training output data and may be processed to generate one or more non-invasive blood pressure measurements to be included in the training input data.

In some examples, the first trained machine-learning algorithm may be trained to synthesize a continuous blood pressure signal using a plethysmography signal and an electrocardiography signal. In other words, the first set of physiological data may comprise a plethysmography signal and an electrocardiography signal, and the second set of physiological data may comprise a continuous blood pressure signal. The subject-specific training data may, for example, be generated using historical physiological data comprising an invasively-acquired continuous blood pressure signal, a plethysmography signal, and an electrocardiography signal. The plethysmography signal and the electrocardiography in the historical physiological data may be included in the training input data, and the invasively-acquired continuous blood pressure signal may be included in the training output data.

In some examples, the first trained machine-learning algorithm may be trained to synthesize a continuous blood pressure signal using a plethysmography signal, an electrocardiography signal and one or more non-invasive blood pressure measurements (e.g. acquired using a pressure cuff). In other words, the first set of physiological data may comprise a plethysmography signal, an electrocardiography signal and one or more non-invasive blood pressure measurements and the second set of physiological data may comprise a continuous blood pressure signal. The subject-specific training data may, for example, be generated using historical physiological data comprising an invasively-acquired continuous blood pressure signal, a plethysmography signal, and an electrocardiography signal. The plethysmography signal and the electrocardiography signal in the historical physiological data may be included in the training input data. Additionally or alternatively, one or more metrics may be determined using the plethysmography signal and/or the electrocardiography signal in the historical physiological data, and the one or more metrics may be included in the training input data (either instead of or in addition to the signal(s) from which the one or more metrics were determined). The one or more metrics may include one or more fiducial points, such as an R-peak in the electrocardiography signal and/or a foot of the plethysmography signal, and/or a metric derived from one or more fiducial points (for instance, a pulse arrival time may be determined from the R-peak in the electrocardiography signal and the foot of the plethysmography signal, assuming the plethysmography signal and the electrocardiography signal are synchronized). The invasively-acquired continuous blood pressure signal may be included in the training output data and may be processed to generate one or more non-invasive blood pressure measurements to be included in the training input data.

In some examples, the first trained machine-learning algorithm may be trained to synthesize a core body temperature using an electrocardiography signal and a respiration waveform (which may be obtained using a capnography signal or the electrocardiography signal). In other words, the first set of physiological data may comprise an electrocardiography signal and a respiration waveform, and the second set of physiological data may comprise a core body temperature. The subject-specific training data may, for example, be generated using historical physiological data comprising a core body temperature and an electrocardiography signal. The core body temperature in the historical physiological data may be included in the training output data. The electrocardiography signal may be included in the training input data and may be processed to generate a respiration waveform to be included in the training input data. Alternatively, the subject-specific training data may be generated using historical physiological data comprising a core body temperature, an electrocardiography signal and a capnography signal. The core body temperature in the historical physiological data may be included in the training output data. The electrocardiography signal may be included in the training input data, and the capnography signal may be processed to generate a respiration waveform to be included in the training input data.

In some examples, the first trained machine-learning algorithm may be trained to synthesize any detected arrhythmia events using a plethysmography signal. In other words, the first set of physiological data may comprise a plethysmography signal, and the second set of physiological data may comprise any detected arrhythmia events. The subject-specific training data may, for example, be generated using historical physiological data comprising a plethysmography signal and an electrocardiography signal. The plethysmography signal in the historical physiological data may be included in the training input data, and the electrocardiography signal may be processed to detect any arrhythmia events to be included in the training output data.

In some examples, the first trained machine-learning algorithm may be trained to synthesize a heart rate and a respiration rate using acceleration data (e.g. acquired by a body accelerometer). In other words, the first set of physiological data may comprise acceleration data, and the second set of physiological data may comprise a heart rate and a respiration rate. The subject-specific training data may, for example, be generated using historical physiological data comprising an electrocardiography signal and acceleration data. The acceleration data in the historical physiological data may be included in the training input data, and the electrocardiography signal may be processed to generate a heart rate and a respiration rate to be included in the training output data. Alternatively, the subject-specific training data may be generated using historical physiological data comprising a plethysmography signal and acceleration data. The acceleration data in the historical physiological data may be included in the training input data, and the plethysmography signal may be processed to generate a heart rate and a respiration rate to be included in the training output data.

In some examples, the first trained machine-learning algorithm may be trained to synthesize one or more motion-filtered physiological signals and/or metrics using corresponding physiological signals and/or metrics that are not motion-filtered and acceleration data (e.g. acquired by a body accelerometer). In other words, the first set of physiological data may comprise acceleration data and one or more physiological signals and/or metrics that are not motion-filtered acquired in a reduced monitoring scenario (such as telemetry monitoring), and the second set of physiological data may comprise one or more motion-filtered physiological signals and/or metrics corresponding to the one or more physiological signals and/or metrics in the first set of physiological data. The subject-specific training data may, for example, be generated using historical physiological data comprising acceleration data and one or more physiological signals and/or metrics acquired in an extended monitoring scenario (such as bedside monitoring). For instance, the one or more motion-filtered physiological signals and/or metrics may comprise one or more cardiac metrics (such as heart rate and/or any detected arrhythmias); the first set of physiological data may then comprise acceleration data and a plethysmography signal. The subject-specific training data may be generated using historical physiological data comprising the one or more cardiac metrics (determined, for instance, using an electrocardiography signal), a plethysmography signal and acceleration data.

It is to be understood that, while the above examples describe the processing of historical physiological data for the subject 110 to generate the subject-specific training data, physiological data for a plurality of individuals having one or more predefined characteristics in common with the subject may be processed in the same way to generate suitable subject-specific training data.

In some examples, the system 100 may further comprise a display device 160, and the first processing system 130 may be configured to control the display device to display the second set of physiological data. In some examples, the first processing system may be configured to control the display device to display both the first set of physiological data and the second set of physiological data.

In some examples, the first processing system 130 may be further configured to determine a quality metric for the second set of physiological data. In some examples, the quality metric may be determined using the first trained machine-learning algorithm; in other words, the first trained machine-learning algorithm may be trained to output both the second set of physiological data and the quality metric. In other examples, a separate machine-learning algorithm may be trained to output the quality metric. A machine-learning algorithm (i.e. the first trained machine-learning algorithm or a separate machine-learning algorithm) may be trained to output the quality metric using the difference between the physiological data output by the first trained machine-learning algorithm during the training process and the corresponding known training output data. The quality metric may be provided to a user or used to determine whether to adjust how the second set of physiological data is generated, as described below.

In some examples, the system 100 may comprise a feedback unit configured to provide one or more user-perceptible outputs to a user (e.g. a clinician). The feedback unit may, for example, comprise the display device 160; the feedback unit may additionally or alternatively comprise any device capable of providing a suitable user-perceptible output. For instance, the feedback unit may be configured to provide one or more of: an image, a light, a textual display, a sound and/or a vibration. The first processing system 130 may be configured to control the feedback unit to provide a user-perceptible output in response to a determination that the second set of physiological data is less reliable than is desired.

For instance, in examples in which a quality metric is determined for the second set of physiological data, the first processing system may be configured to control the feedback unit to provide a first user-perceptible output in response to the quality metric failing to exceed a predetermined quality threshold. The value of the predetermined quality threshold may depend on an application in which the first trained machine-learning algorithm is being used. In some examples, the value of the predetermined quality threshold may be defined based on a user input.

In some examples, the first processing system 130 may be configured to obtain one or more conditions under which the first set of physiological data is expected to result in an unreliable second set of physiological data. The first processing system may then be configured to control the feedback unit to provide a second user-perceptible output in response to at least one of the one or more conditions being met.

The one or more conditions under which the first set of physiological data is expected to result in an unreliable second set of physiological data may be identified by the second processing system 150 during training of the first trained machine-learning algorithm, by identifying any conditions under which the output generated by the first machine-learning algorithm differs from the corresponding training output data by more than a predetermined amount. The one or more conditions may, for example, include a condition that a signal or metric in the first set of physiological data (or derivable from the first set of physiological data) is above a threshold value, below a threshold value or falls within a defined range. For instance, where the first trained machine-learning algorithm is trained to generate a respiration rate based on a plethysmography signal and an electrocardiography signal, it may be identified during training of the first trained machine-learning algorithm that the respiration rate generated by the first trained machine-learning algorithm is unreliable for a particular heart rate range and/or when particular arrhythmias are detected. The first processing system may then be configured to control the feedback unit to provide the second user-perceptible output in response to a heart rate determined using the first set of physiological data falling within the particular heart rate range and/or in response to the particular arrhythmias being detected using the first set of physiological data.

In some examples, the first processing system 130 may be configured to obtain the subject-specific training data used to train the first machine-learning algorithm. If the same processing system is used to train the first machine-learning algorithm and to generate the second set of physiological data, the step of obtaining the subject-specific training data will have been performed by the first processing system 130 during the training process. The first processing system may then be configured to compare the subject-specific training data with the first set of physiological data to determine a measure of difference, and to control the feedback unit to provide a third user-perceptible output in response to the measure of difference exceeding a predetermined difference threshold. The value of the predetermined difference threshold may depend on an application in which the first trained machine-learning algorithm is being used. In some examples, the value of the predetermined difference threshold may be defined based on a user input.

In some examples, the first processing system 130 may be configured to, in response to a predetermined condition being met, process the first set of physiological data, using a second trained machine-learning algorithm, to generate the second set of physiological data. The second trained machine-learning algorithm is a machine-learning algorithm trained using second training data that is less subject-specific (i.e. more generic) than the subject-specific training data used to train the first trained machine-learning algorithm. For instance, where the subject-specific training data comprises only historical physiological data for the subject 110 (i.e. where the first machine-learning algorithm has been trained using only historical physiological data for the subject, or where the first machine-learning algorithm has been trained by tuning a generic machine-learning algorithm using only historical physiological data for the subject), the second trained machine-learning algorithm may be trained using physiological data for each of a plurality of individuals having a same one or more characteristics as the subject. Where the subject-specific training data comprises physiological data for each of a plurality of individuals having a same one or more characteristics as the subject, the second trained machine-learning algorithm may be trained using generic training data.

Where the first machine-learning algorithm was trained using historical physiological data for the subject, the predetermined condition may, for example, be a condition that more than a predetermined amount of time has passed since the historical physiological data was acquired. In other words, the first processing system 130 may be configured to process the first set of physiological data using the first trained machine-learning algorithm until the predetermined amount of time has passed, after which the second trained machine-learning algorithm is used instead. After the predetermined amount of time, it may be expected that physiological data for the subject is sufficiently different to the training data that a less subject-specific machine-learning algorithm would provide a more accurate result. The predetermined amount of time may be based on an average deterioration/recovery curve for the type of subject and the type of condition (e.g. based on literature data).

In some examples, where the first processing system 130 is configured to determine a quality metric for the second set of physiological data, the predetermined condition may be a condition that the quality metric is lower than an expected quality of the second trained machine-learning algorithm. The expected quality of the second trained machine-learning algorithm may be an output of the second trained machine-learning algorithm (or an output of a separate machine-learning algorithm); thus, the first processing system 130 may process the first set of physiological data using both the first trained machine-learning algorithm and the second trained machine-learning algorithm in parallel, and which output is used as the second set of physiological data may depend on which machine-learning algorithm has the higher quality metric.

In some examples, the predetermined condition may relate to a difference between the subject-specific training data and the first set of physiological data. For instance, a measure of difference between the subject-specific training data and the first set of physiological data may be determined as described above, and the first processing system 130 may be configured to process the first set of physiological data using the second trained machine-learning algorithm in response to the measure of difference exceeding a predetermined threshold. For example, the first set of physiological data may be compared, for each signal/metric in the first set, to a range of values present in the subject-specific training data for the signal/metric. The first processing system 130 may be configured to process the first set of physiological data using the second trained machine-learning algorithm in response to first set of physiological data including data values that fall outside the range of values present in the subject-specific training data (or that fall outside the range of values by more than a predetermined amount).

In some examples, the first processing system 130 may be configured to process the first set of physiological data using both the first trained machine-learning algorithm and the second trained machine-learning algorithm. The second set of physiological data may then be generated by determining a weighted average of the outputs of the first trained machine-learning algorithm and the second trained machine-learning algorithm. The weighted average may, for example, be weighted according to time, with a weight given to the output of the second trained machine-learning algorithm increasing over time. In another example, a quality metric may be determined for each output, and the weighted average may be weighted according to the quality metrics.

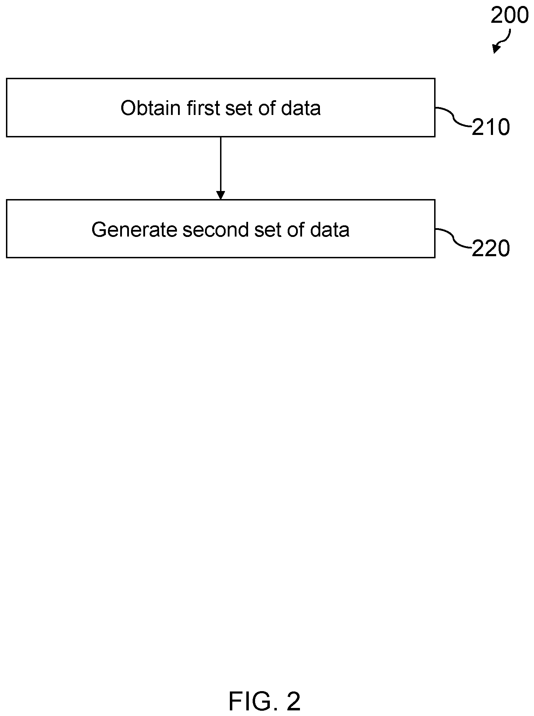

Fig. 2 illustrates a computer-implemented method 200 for monitoring a subject, according to an embodiment of the invention.

The computer-implemented method 200 begins at step 210, at which a first set of physiological data for the subject is obtained.

At step 220, the first set of physiological data is processed, using a first trained machine-learning algorithm, to generate a second set of physiological data for the subject. The first trained machine-learning algorithm is a machine-learning algorithm trained using subject-specific training data comprising physiological data corresponding to the first set of physiological data and physiological data corresponding to the second set of physiological data. The subject-specific training data may comprise historical physiological data for the subject and/or physiological data for each of a plurality of individuals having a same one or more predefined characteristics as the subject, as described above.

It will be understood that the disclosed methods are computer-implemented methods. As such, there is also proposed a concept of a computer program comprising code means for implementing any described method when said program is run on a processing system.

The skilled person would be readily capable of developing a processing system for carrying out any herein described method. Thus, each step of a flow chart may represent a different action performed by a processing system, and may be performed by a respective module of the processing system.

As discussed above, the system makes use of a processing system to perform the data processing. The processing system can be implemented in numerous ways, with software and/or hardware, to perform the various functions required. The processing system typically employs one or more microprocessors that may be programmed using software (e.g. microcode) to perform the required functions. The processing system may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processing system may be associated with one or more storage media such as volatile and non-volatile computer memory such as RAM, PROM, EPROM, and EEPROM. The storage media may be encoded with one or more programs that, when executed on one or more processing systems and/or controllers, perform the required functions.

Various storage media may be fixed within a processing system or controller may be transportable, such that the one or more programs stored thereon can be loaded into a processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processing system may be implemented by a single processing system or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Ordinal numbers (e.g. "first", "second" and so on) have been used purely to distinguish different elements from one another for the sake of clarity, and does not necessarily imply a specific order, importance, relationship, or presence of all numbered elements. Reference to a non-"first" (e.g. "second" or "third") element does not necessitate that a "first" element be present. The skilled person would be capable of relabeling any such elements as appropriate (e.g. relabeling a "second" element as a "first" element if only the second element is present).

Any reference signs in the claims should not be construed as limiting the scope.

## Claims

1. A processing system (130) for monitoring a subject (110), the processing system being configured to:
obtain a first set of physiological data (125) for the subject; and
process the first set of physiological data, using a first trained machine-learning algorithm (155), to generate a second set of physiological data for the subject,
wherein the first trained machine-learning algorithm is a machine-learning algorithm trained using subject-specific training data comprising physiological data corresponding to the first set of physiological data and physiological data corresponding to the second set of physiological data.

2. The processing system (130) of claim 1, wherein the subject-specific training data comprises historical physiological data (145) for the subject (110).

3. The processing system (130) of claim 1 or 2, wherein the subject-specific training data comprises physiological data for each of a plurality of individuals, wherein each of the plurality of individuals have a same one or more predefined characteristics as the subject.

4. The processing system (130) of claim 3, wherein the one or more predefined characteristics includes a medical condition.

5. The processing system (130) of any of claims 1 to 4, wherein the processing system is further configured to determine a quality metric for the second set of physiological data.

6. The processing system (130) of claim 5, wherein the processing system is further configured to control a feedback unit to provide a first user-perceptible output in response to the quality metric failing to exceed a predetermined quality threshold.

7. The processing system (130) of any of claims 1 to 6, wherein the processing system is further configured to:
obtain one or more conditions under which the first set of physiological data (125) is expected to result in an unreliable second set of physiological data; and
control a feedback unit to provide a second user-perceptible output in response to at least one of the one or more conditions being met.

8. The processing system (130) of any of claims 1 to 7, wherein the processing system is further configured to:
obtain the subject-specific training data used to train the first machine-learning algorithm (155);
compare the subject-specific training data with the first set of physiological data (125) to determine a measure of difference; and
control a feedback unit to provide a third user-perceptible output in response to the measure of difference exceeding a predetermined difference threshold.

9. The processing system (130) of any of claims 1 to 8, wherein:
the processing system is further configured to, in response to a predetermined condition being met, process the first set of physiological data (125), using a second trained machine-learning algorithm, to generate the second set of physiological data,
wherein the second trained machine-learning algorithm is a machine-learning algorithm trained using second training data that is less subject-specific than the subject-specific training data used to train the first trained machine-learning algorithm.

10. The processing system (130) of any of claims 1 to 9, wherein the processing system is further configured to control a display device (160) to display the second set of physiological data.

11. The processing system (130) of any of claims 1 to 10, wherein the first set of physiological data (125) comprises an 8-lead electrocardiography signal and the second set of physiological data comprises a 12-lead electrocardiography signal.

12. The processing system (130) of any of claims 1 to 11, wherein the first set of physiological data (125) comprises an electrocardiography signal and the second set of physiological data comprises a respiration rate.

13. The processing system (130) of any of claims 1 to 12, wherein the first set of physiological data (125) comprises a plethysmography signal and the second set of physiological data comprises a respiration rate.

14. A computer-implemented method (200) for monitoring a subject, the computer-implemented method comprising:
obtaining a first set of physiological data (125) for the subject; and
processing the first set of physiological data, using a first trained machine-learning algorithm (155), to generate a second set of physiological data for the subject,
wherein the first trained machine-learning algorithm is a machine-learning algorithm trained using subject-specific training data comprising physiological data corresponding to the first set of physiological data and physiological data corresponding to the second set of physiological data.

15. A computer program product comprising computer program code means which, when executed on a computing device having a processing system, cause the processing system to perform all of the steps of the method (200) according to claim 14.
